# EUROPEAN PATENT APPLICATION

(11) **EP 4 321 521 A1**
(43) Date of publication of application: **14.02.2024**
(21) Application number: 22190074.9
(22) Date of filing: 11.08.2022
(51) Int. Cl.: C07K 1/14, C07K 1/30, C07K 1/36

(54) **FRACTIONATION OF PROTEINS FOR PROTEOMICS**

(71) Applicant: PreOmics GmbH, 82152 Planegg/Martinsried (DE)
(72) Inventor: KULAK, Nils A, 80686 München (DE)
(74) Representative: Dentons Patent Solutions Rechtsanwaltsgesellschaft mbH

(57) **Abstract**

The present invention relates to a method of fractionating a sample comprising proteins, polypeptides and/or peptides, said method comprising (a) performing a first change of physicochemical conditions of said sample, wherein said first change establishes precipitating conditions for said proteins, polypeptides and/or peptides, but does not establish denaturing conditions for said proteins, polypeptides and/or peptides; and (b) performing one or both of the following (i) and (ii): (i) adding solid particulate matter to said sample; and (ii) performing said method in a vessel with a rough surface; wherein steps (a) and (b) can be effected concomitantly or in any order and yield a first fraction of proteins, polypeptides and/or peptides as a first precipitate on said particulate matter and/or on said inhomogeneous surface, and a second fraction of proteins, polypeptides and/or peptides remaining in a supernatant; and (c) subjecting said first precipitate to a second change of physicochemical conditions which are capable of solubilizing a part of said first precipitate, thereby obtaining a third fraction remaining precipitated and a fourth fraction in solution.

## Description

This invention relates to a method of fractionating a sample comprising proteins, polypeptides and/or peptides, said method comprising (a) performing a first change of physicochemical conditions of said sample, wherein said first change establishes precipitating conditions for said proteins, polypeptides and/or peptides, but does not establish denaturing conditions for said proteins, polypeptides and/or peptides; and (b) performing one or both of the following (i) and (ii): (i) adding solid particulate matter to said sample; and (ii) performing said method in a vessel with a rough surface; wherein steps (a) and (b) can be effected concomitantly or in any order and yield a first fraction of proteins, polypeptides and/or peptides as a first precipitate on said particulate matter and/or on said inhomogeneous surface, and a second fraction of proteins, polypeptides and/or peptides remaining in a supernatant; and (c) subjecting said first precipitate to a second change of physicochemical conditions which are capable of solubilizing a part of said first precipitate, thereby obtaining a third fraction remaining precipitated and a fourth fraction in solution.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Polypeptide/protein fractionation, separation, or depletion remains a challenge in biopharmaceutical research. For example, one of the major goals in current pharmaceutical research is the identification, detection and quantitation of polypeptide/protein biomarkers out of body fluids such as blood plasma, also referred to as "liquid biopsies". Because of the complexity of the samples and the very high dynamic range of polypeptides/proteins within bodily fluids, low abundant polypeptides/proteins are often difficult or in many instances impossible to detect. For this purpose, most current methods aim to deplete and remove high abundant polypeptides/proteins such as serum albumin and immunoglobulins by selective binding to proteins which have a higher affinity for the respective protein. For example, Protein-G beads are commonly employed to reduce the quantity of IgG protein in the sample of interest, and cartridges are available, for example from Thermo Fisher (High Select Top14) and Agilent (Multiple Affinity Removal Spin Cartridge Human 14). Furthermore, fractionation methods such as ion-exchange chromatography are also often used to separate polypeptides/proteins and reduce the complexity and dynamic range of the sample.

The concept of selective protein/polypeptide precipitation is well established and was described previously (E. J. Cohn, L. E. Strong, W. L. Hughes, D. J. Mulford, J. N. Ashworth, M. Melin, and H. L. Taylor; Journal of the American Chemical Society 1946 68 (3), 459-475; DOI: 10.1021/ja01207a034). Here the pH, temperature, and ethanol content are utilized to induce a step-wise precipitation of certain polypeptides/proteins in solution in blood plasma. This process remains one of the major processes in large-scale protein enrichment in the pharmaceutical industry to purify certain protein/polypeptides species. This process however requires large amounts of starting material to generate a sufficient precipitate that can be filtered or spun down in order to successfully separate the precipitated polypeptides/proteins from the remaining soluble polypeptides/proteins. Furthermore, automatizing filtration or centrifugation remains challenging.

In view of shortcomings of the state of the art, the technical problem underlying the present invention can be seen in the provision of improved means and methods for sample processing or sample fractionation, in particular of samples comprising polypeptides or in the field of proteomics including research, manufacturing and diagnostics.

This technical problem has been solved by the enclosed claims and as evidenced by the Example comprised in this disclosure.

Accordingly, the present invention, in a first aspect, relates to a method of fractionating a sample comprising proteins, polypeptides and/or peptides, said method comprising (a) performing a first change of physicochemical conditions of said sample, wherein said first change establishes precipitating conditions for said proteins, polypeptides and/or peptides, but does not establish denaturing conditions for said proteins, polypeptides and/or peptides; and (b) performing one or both of the following (i) and (ii): (i) adding solid particulate matter to said sample; and (ii) performing said method in a vessel with a rough surface; wherein steps (a) and (b) can be effected concomitantly or in any order and yield a first fraction of proteins, polypeptides and/or peptides as a first precipitate on said particulate matter and/or on said inhomogeneous surface, and a second fraction of proteins, polypeptides and/or peptides remaining in a supernatant; and (c) subjecting said first precipitate to a second change of physicochemical conditions which are capable of solubilizing a part of said first precipitate, thereby obtaining a third fraction remaining precipitated and a fourth fraction in solution.

Related thereto, the present invention provides a method of fractionating a sample comprising biomolecules, said method comprising (a) performing a first change of physicochemical conditions of said sample, wherein said first change establishes precipitating conditions for said biomolecules, but does not establish denaturing conditions for said biomolecules; and (b) performing one or both of the following (i) and (ii): (i) adding solid particulate matter to said sample; and (ii) performing said method in a vessel with a rough surface; wherein steps (a) and (b) can be effected concomitantly or in any order and yield a first fraction of biomolecules as a first precipitate on said particulate matter and/or on said inhomogeneous surface, and a second fraction of biomolecules remaining in a supernatant; and (c) subjecting said first precipitate to a second change of physicochemical conditions which are capable of solubilizing a part of said first precipitate, thereby obtaining a third fraction remaining precipitated and a fourth fraction in solution.

Preferred biomolecules are biological macromolecules. Preferred biological macromolecules, in addition to the above disclosed proteins, polypeptides and peptides, include nucleic acids, saccharides and lipids.

The term "fractionating" has its art-established meaning. It refers to dividing a mixture of compounds or analytes into at least two separate mixtures, wherein said separate mixtures differ in their relative contents with regard to at least one of said compounds or analytes. In other words, at least one analyte is enriched in at least one fraction as compared to at least one other fraction. Fractionating may lead to at least one analyte being substantially or totally depleted from at least one fraction, but does not have to. In a more general sense, fractionation may lead to a modified or decreased dynamic range or complexity as compared to the original mixture, e.g. the ratio of the amount of most abundant analyte to the amount of the least abundant analyte may be decreased in at least one of the obtained fractions as compared to the original mixture. Such decrease in dynamic range will generally increase the amount of analytes amenable to detection in subsequent analytical procedures, e.g. mass spectrometry (MS). The term "complexity" as used here refers to the number of analytes, in particular biomolecules, more specifically proteins, polypeptides and or peptides comprised in a given fraction. A compression of the dynamic range generally entails a higher number of analytes being detected.

The terms "protein", "polypeptide" and "peptide" have their art-established meanings as well. In particular, peptides and polypeptides are polycondensates of amino acids, preferably of the proteinogenic L-alpha-amino acids. Herein, such polycondensate is referred to as peptide if it contains 30 or less amino acids, and longer polycondensates are referred to as polypeptides. Preferred peptides are those with a length from 20 to 30 amino acids. Generally, peptides and polypeptides are single chains, which does not exclude the presence of cross-links such as disulfides and/or modifications such as phosphorylation. Proteins on the other hand may have a more complex structure in that they may be oligomers of polypeptides and/or comprise non-proteinaceous components such as prosthetic groups. The analysis of such proteins, polypeptides and peptides is of particular interest when it comes to distinguishing healthy from diseased states at a molecular level.

"Physicochemical conditions" embrace both physical parameters such as temperature as well as chemical parameters such as solvent composition. Physicochemical conditions have an influence on the state of analytes in a mixture which state may be expressed in terms of the chemical potential of an analyte.

The change in physicochemical conditions referred to as "first change" is a means to trigger precipitation of one or more analytes in a mixture - to a larger or lesser extent. The changed conditions favor the precipitated state of the analyte. In that respect, a line has to be drawn between precipitation and denaturation. As discussed in more detail further below, the invention employs conditions which trigger precipitation, but avoid denaturation.

The term "precipitation" has its art-established meaning, in particular as used in the field of chemistry. It refers to a change of state of the composition of matter under consideration (here biomolecules including proteins, polypeptides, and/or peptides) from dissolved or soluble to insoluble. The insoluble matter may initially form as a suspension which in turn may sediment, for example under the influence of gravity (which may be enhanced by procedures such as centrifugation) to yield a precipitate (insoluble matter in a particular region, generally the bottom of the vessel or container used). In case of centrifugation, the precipitate is commonly referred to as pellet.

Said processing or fractionating up to and including step (b) yields one or more first fractions of proteins, polypeptides and/or peptides as a precipitate on said particulate matter and/or said inhomogeneous surface, and a second fraction of proteins, polypeptides and/or peptides remaining in a supernatant. Such fractionation is in principle inherent to the method of the first aspect in its broadest definition. It yields a supernatant and a precipitate which terms both have their art-established meanings. After processing in accordance with the method of the first aspect, a part of the proteins, polypeptides and peptides of the original sample has changed its state of matter and is no longer in solution but adheres - as precipitate - to said inhomogeneous surface and/or to said solid particulate matter. The remainder of the analytes is still in solution and constitutes the supernatant.

This introduces the notion of at least one first fraction and a second fraction. More than one first fraction, i.e., more than one precipitate may be obtained because the method of the invention may employ more than one type of particulate matter and/or more than one type of inhomogeneous surface. Preferred is one type of particulate matter or inhomogeneous surface.

Precipitation occurs when a solution is super-saturated, i.e., the solution contains more solute than what can stably be maintained in solution under the conditions given. Yet, a super-saturated state may persist for certain amounts of time. This is because for precipitation to occur, generally a process known as nucleation has to take place. If nucleation does not take place, the super-saturated state may become metastable. "Metastable" refers to a state which is stable in response to slight changes of conditions, but instable when larger changes of conditions occur. Nucleation may be facilitated by seeding. Seeding generally converts a metastable super-saturated state into an instable one which entails precipitation. Seeding, as will become apparent further below, is a key feature of the present invention in mechanistic terms.

Physicochemical conditions have been described as being suitable triggers of precipitation; see the literature cited in the background section above. A listing of preferred physicochemical conditions to be changed in accordance with the invention is subject of preferred embodiments disclosed further below. The term "harsh" in relation to conditions as used herein refers to physicochemical conditions which are particularly apt to trigger precipitation. Harsh conditions, in extreme cases, may cause precipitation of all or substantially all protein(s), polypeptide(s) and/or peptide(s) from said sample. For that reason, harsh conditions are generally less preferred. Another disadvantage of harsh conditions is that, in addition to precipitation, they may trigger denaturation of biomolecules such proteins, polypeptides and peptides which are preferred analytes. As discussed in more detail below, means (b) in accordance with the invention allow to use less harsh conditions including conditions which in the absence of means (b) could not be employed because they would not trigger precipitation at all or to an insufficient extent. Preferred less harsh conditions are non-denaturing conditions as well as those specific conditions which are disclosed further below.

Indeed, the present inventors surprisingly discovered that the effect of a change of physicochemical conditions may be modulated or enhanced - this is expressed in step (b) of the method of the first aspect of the invention. Such modulation or enhancement may, but does not have to, affect different analytes to a different extent. The measures in accordance with step (b) are physical in nature. Their overarching principle is a structured or rough surface. Such structured surface may be embodied as particulate matter to be added to the sample to be processed or fractionated, or as a modification of the surface of the vessel where processing or fractionating takes place.

The structured surface is not particularly limited. It surprisingly turned out that the composition of the particulate matter is irrelevant. Moreover, the structured surface of the vessel may be made of the same material (glass, plastic etc.) as the remainder of the vessel.

The surface of the vessel may be rough throughout or in one or more regions. The size of said one or more regions is macroscopic, wherein as the structural inhomogeneity or roughness in the rough regions is microscopic. For example, the region may have a diameter of 1 to 20 mm such as 2 to 10 mm including 3, 4, 5, 6, 7, 8, or 9 mm. On the other hand, the structural inhomogeneity or roughness is on a nanometer to µm scale, preferably between 0.4 and 100 µm, more preferably between 1 and 60 µm such as 2 to 30 µm including 3, 4, 5, 6, 7, 8, 9, 10, 15, 20 and 25 µm.

Structural inhomogeneity or roughness of a surface may be described in terms of the roughness parameters Ra. Ra is the arithmetical mean deviation from the center line of a profile along the surface under consideration. The dimension of Ra is length; it is usually specified in µm. In a preferred embodiment, the above given preferred values for roughness are Ra values. Roughness may be determined by instruments such as profilometers, optical comparators, interferometers and microscopes. A preferred instrument is a profilometer.

Generally, the preferred scale of structural inhomogeneity is also the preferred size of said solid particulate matter.

Without wishing to be bound by a particular theory, it is considered that said solid particulate matter and said rough surface stabilize very small nuclei (the initial intermediates of a precipitation process) which always form but would dissolve again in the absence of particles or a rough surface. To explain further, small nuclei have a larger surface to volume ratio as compared to larger nuclei. The free energy of a nucleus depends on both its surface and its volume. In accordance with classical nucleation theory, the surface term is always positive and disfavors nucleation such that the total free energy of nucleation is positive for small nuclei. Only with growing nucleus size, the volume term dominates and eventually nucleation proceeds and precipitation occurs. It is considered that said particulate matter or said rough surface modifies the surface energy of small nuclei such that dominance of the volume term occurs already for very small nuclei.

In accordance with step (c) of the method of the first aspect, a second change of physicochemical conditions is performed. The purpose of step (c) is to sub-fractionate the precipitate resulting from steps (a) and (b). The conditions established by said second change are solubilizing such that a part of said precipitate is brought into solution giving rise to a fourth fraction, while the remainder remains precipitated and defines a third fraction. Of note, step (c) is performed such that partial solubilization occurs. In other words, it yields non-empty third and fourth fractions which in turn serves to further reduce complexity of the sample and compress the dynamic range of the analytes. Preference is given to non-denaturing solubilization. A preferred class of agents in this respect are surfactants, in particular mild or non-denaturing surfactants which are subject of preferred embodiments discussed further below. In the Example, the non-denaturing surfactant SDC is used and provides for unprecedented depth of protein identification in plasma. Having said that, the invention provides for further, additional or alternative means of partial solubilization; for details see below.

Generally, the method of the invention performs superior when compared to established methods in terms of reducing the dynamic range of analytes in a sample and/or increasing the number of analytes amenable to detection. Reducing dynamic range improves the performance of any downstream analytical method such as mass spectrometry. Analytical methods such as mass spectrometry are inherently characterized by a limited dynamic range within which satisfactory qualitative and quantitative detection is possible. By reducing the dynamic range of the analytes comprised in a sample, e.g. by reducing the relative amount of the most abundant analytes, low abundant analytes which in a sample which has not been processed with the method of the invention would escape detection, become detectable and quantifiable. Of note, already the combination of steps (a) and (b) provides for such improvement. Surprisingly, as stated above, by performing step (c) in addition a yet greater depth of proteome analysis is achieved.

The method of the invention furthermore offers a dramatic improvement in processing speed as compared to art-established methods of protein or polypeptide fractionation. While processing over night or for days is common in the art, and also envisaged in accordance with the invention (any processing time between 30 sec and over night (about 12 hrs) or 1 week being preferred), the method of the invention more preferably can be performed on a time scale to be measured in minutes, for example 1 min to 3 hrs, 2 to 60 min, such as 3, 4, 5, 6, 7, 8, 9, 10, 15, 20, 25, 30, 40 or 50 minutes.

A further improvement is the amount of sample sufficient for successful performance of the method. While prior art methods require generally large amounts or even liters of bodily fluids, the present invention works with small sample sizes such as 1 to 1000 µl, 5 to 100 µl such as 10, 20, 30, 40, 50, 60 or 80 µl. In case of bodily fluids with low protein/polypeptide/peptide concentrations such as urine, also larger sample sizes are considered in addition to the ranges and values specified above, such as 1 µl to 100 ml or 10 µl to 50 ml.

A further advantage of the invention, in particular of the modulating/enhancing effect of an inhomogeneous surface or particulate matter on precipitation is that the physicochemical conditions may be employed in a manner which is less harsh as compared to the art-established procedures. In other words, in the presence of measures in accordance with step (b) of the first aspect, physicochemical conditions may be changed to a lesser extent as compared to the absence of said measures while yielding still satisfactory results in terms of both precipitation and fractionation. Indeed, less strongly precipitating conditions are found to be not only satisfactory, but superior to separate low abundant from high abundant proteins, polypeptides or peptides, e.g. in plasma samples. While strongly precipitating conditions (such as those described in Cohn et al., loc. cit.) generally provide for less selective precipitation of a mixture of protein, polypeptides and/or peptides, less precipitating conditions were surprisingly found to provide for more selective precipitation in the context of seeded precipitation as implemented by steps (a) and (b). Of note, it is envisaged or even preferred to precipitate only a fraction of the total protein(s), polypeptide(s) and/or peptide(s) in said sample. Doing so modifies the dynamic range and/or sample complexity, thereby increasing the number of detectable protein(s), polypeptide(s) and/or peptide(s). Said fraction may be in the range from 0.01 to 90% by weight, more preferably between 0.1 and 60% by weight such as 0.5 to 50% by weight.

Since, as shown in the Example, the method of the invention allows the detection of a larger number of protein(s), polypeptide(s) and/or peptide(s) in a given sample (in other words, the proteome is detected with a greater depth), said method provides for novel markers and novel marker combinations for a given state of a cell, tissue or organism ("state" including healthy and diseased), which novel markers in turn provide for improved diagnostics. Given that in particular the detection of protein is of interest in many analytical or diagnostic applications, proteins are the especially preferred analytes.

In a preferred embodiment, said proteins, polypeptides and/or peptides are dissolved in said sample.

In a further preferred embodiment, said sample is a cell lysate or a bodily fluid, wherein optionally said bodily fluid or cell lysate has been treated with an anticoagulant.

Treatment of biological sample with anticoagulants is common in the art, in particular when it comes to clinical samples. To give an example, while serum is the liquid component of blood which may coagulate when stored, the addition of anticoagulants serves to prevent coagulation and gives rise to plasma. The blood clot in case of serum and the blood cells in case of plasma are generally removed by centrifugation. Anticoagulants are known in the art, wherein EDTA is particularly common, EDTA K2 and EDTA K3 being preferred.

The application of the methods of the invention to a cell lysate extends to what is known in the art as "host cell protein (HCP)" analysis. HCP analysis refers to the monitoring of the purification of a biopharmaceutical product, e.g. an antibody, from the cells, e.g. immortalized B-cells, used for its production/manufacturing. Such purification is generally a stepwise process with host cell proteins being depleted and finally yielding a pharmaceutical grade product. Said host cell proteins include potentially allergenic or otherwise undesirable proteins or polypeptides from the host cell. In other words, HCP analysis may be implemented using the method of the invention.

Bodily fluids include blood, serum, plasma, sputum, nasal swab, urine, vaginal fluid, semen, and cerebrospinal fluid. Cell lysates may be obtained from any cell, tissue or biopsy. Preferably, insoluble parts of said cell lysate are removed, e.g. by centrifugation or filtration prior to performing the method of the invention.

The term "antibody" has its art-established meaning. It embraces all naturally occurring classes and subclasses such as IgG (including IgG1, IgG2, IgG3 and IgG4), IgA, IgM, IgD and IgE as well as camelid antibodies. As used herein, the term "antibody" furthermore embraces fragments (such as Fab) and engineered antigen-binding molecules such as single-chain antibodies, bi- or multispecific antibodies and any antigen-binding molecule with at least three (especially in case of camelid antibodies and their derivatives), preferably six complementarity determining regions (CDRs).

In a further preferred embodiment, step (a), step (b), or both steps (a) and (b) are performed more than once, preferably with the supernatant as defined above. E.g. once a first precipitate has been removed, steps (a) and (b) may be repeated with the supernatant under the same or with modified physicochemical conditions and/or with the same or a different inhomogeneous surface or particulate matter. This allows to further fractionate the supernatant obtained upon the first performance of steps (a) and (b).

Similarly, step (c) may independently be performed repeatedly. For example, for the purpose of solubilizing, more than one change of conditions may be performed. In such a case, the solubilizing effect may be, for example, increased in a stepwise manner. This may be achieved by increasing the concentration of the solubilizing agent which preferably is, as disclosed further below, a surfactant, more preferably a non-denaturing surfactant as cholates or deoxycholates including SDC. Other implementations of repeated performance of step (c) may be the use of different solubilizing agents for each performance, for example the use of SDC in one solubilizing step and of OTG in another one.

In a further preferred embodiment, said physicochemical conditions upon said first change and/or said second change (i) do not include temperatures above 70°C, (ii) concentrations of denaturing surfactants such as SDS above 0.8% (w/v), and (iii) concentrations of organic solvents such as acetonitrile of or above 20% (v/v); and/or (iv) are non-denaturing conditions for said proteins, polypeptides and/or peptides.

Commonly used workflows for sample preparation (see introductory part of this specification) involves complete or substantially complete precipitation of proteins, polypeptides and peptides. To this end, quite harsh conditions, in particular those recited in item (i) of the embodiment above are employed. The methods of the invention on the other hand, owing to the use of particles and/or a rough surface, allow to dispense with such harsh conditions.

This provides for distinct advantages. As stated above, proteomes are generally characterized by a large dynamic range. As a consequence, detection of low abundance proteins in the presence of highly abundant proteins such as antibodies and albumins is rendered difficult. Yet, for many applications in the field of research and diagnostics, capturing low abundance proteins is of interest. For example, in diseased conditions, small amount of markers characteristic of the disease leave the affected local tissue and enter the plasma where they become highly diluted. Therefore, compression of the dynamic range is desirable. The use of particulate matter and/or rough surfaces is one means to achieve this. The avoidance of harsh conditions, in particular of denaturing conditions, is a further means. To explain further, comprehensive denaturation and concomitant precipitation generally leave the dynamic range substantially unchanged: all analytes are precipitated and subsequently brought back into solution for further processing. Less harsh conditions (for preferred implementations thereof see below) on the other hand have a more differentiated effect. In particular, experience tells that the mentioned highly abundant proteins have less propensity to precipitate under non-denaturing conditions (they would do so under the conditions of the prior reviewed herein, though) than the mentioned low abundance proteins. As a consequence, precipitation under non-denaturing conditions preferentially, but generally not completely, depletes highly abundant proteins from the precipitate. What is found in the precipitate is therefore still highly informative, but characterized by a compressed dynamic range.

As such, conditions in accordance with the invention are non-denaturing conditions. This applies in particular to steps (a) and (b) and is preferred for step (c).

The terms "conditions" as used herein, if not specified otherwise, refers to the conditions after "changing the physicochemical conditions" in accordance with steps (a) and (c), respectively, of the method of the first aspect. It is understood that the conditions prior to said the first change will generally be non-denaturing, given that said sample comprising proteins, polypeptides and/or peptides comprises said proteins, polypeptides and/or peptides in their native, i.e., non-denatured state, e.g. because said sample has been obtained from its natural biological environment without or substantially without changing its physicochemical conditions, at least not to an extent which entails denaturation.

The term "non-denaturing conditions" in relation to protein, polypeptides and peptides is commonly used in the art and understood by a skilled person. The process of denaturation leads to the loss of a defined three-dimensional structure. Such loss generally impairs function, "function" including binding to a cognate binding partner and/or ability of an enzyme to process its cognate substrate. Moreover, said loss is generally accompanied by an increase in size (measured, e.g., in term of the hydrodynamic radius) as well as by increase of width of the distribution of size.

For the sake of completeness, a preferred definition of the threshold separating non-denaturing from denaturing conditions is as follows.

Dynamic light scattering (DLS) is an analytical method which can be used to determine the size distribution of small particles in suspension or solution by measuring the hydrodynamic radius *R*ₕ of particles through the Stokes-Einstein relation which connects the diffusion constant D with the hydrodynamic radius *R*ₕ. It allows to distinguish non-denatured from denatured states of proteins, polypeptides and peptides. The non-denatured or native state is characterized, as stated above, by a defined structure which gives to a peak of the size distribution which has limited breadth. Denaturation on the other causes the loss of said defined structure, and the previous homogenous ensemble of molecules of which all or substantially all have said defined structure is converted in a more heterogeneous ensemble of disordered structures which share the lack of the native structure, but otherwise are diverse, in particular with regard to their hydrodynamic radius *Rₕ*. This is reflected by a broadening of the peak of the distribution as obtained by DLS, generally accompanied by an increase of the average hydrodynamic radius. Increases of the mean *Rh* as well as broadening of the peak of the distribution of *Rh*, preferably statistically significant changes of either parameter, are both hallmarks of denaturation. The threshold separating native from denatured in accordance with the invention capitalizes on the change of peak width.

A model-independent measure of the breadth of a peak is the full width at half maximum (FWHM). More specifically, the FWHM is the difference between two values of the independent variable at which the dependent variable is equal to half of its maximum value. In case of a Gaussian distribution governing the shape of said peak, FWHM is a simple function of the standard deviation · of said Gaussian distribution.

For the purpose of defining the threshold separating denaturing from non-denaturing conditions, in principle any protein may be used. Preferred proteins in this respect are GAPDH and MTAP, preferably of human origin, more preferably human GAPDH.

Preferred non-denaturing conditions are physiological conditions. The term "physiological conditions has its art-established meaning and refers to intracellular conditions. Preferred physiological conditions are as follows: 14 mM Na⁺, 140 mM K⁺, 10⁻⁷ mM Ca²⁺, 20 mM Mg²⁺, 4 mM Cl⁻, 10 mM HCO₃⁻, 11 mM HPO₄²⁻ and H₂PO₄⁻, 1 mM SO₄²⁻, 45 mM phosphocreatine, 14 mM carnosine, 8 mM amino acids, 9 mM creatine, 1.5 mM lactate, 5 mM ATP, 3.7 mM hexose monophosphate, 4 mM protein and 4 mM urea. A preferred temperature is 37°C. A preferred pressure is 1013.25 hPa.

Accordingly, in a preferred embodiment, a broadening as compared to non-denaturing conditions, preferably a statistically significant broadening, of the peak of the distribution function of the hydrodynamic radius of said protein as determined by dynamic light scattering is indicative of denaturing conditions. A preferred measure of the breadth of said peak is the FWHM.

In a particularly preferred embodiment, a relative increase of said FWHM of at least 50%, at least 40%, at least 30% or at least 20% upon a change in physicochemical conditions is indicative of said change of conditions having led to denaturing conditions. The term relative increase is defined as follows: (FWHM_{f} - FWHMi) x 100 / FWHMi, wherein the subscripts i and f refer to initial (before changing conditions) and final (after changing conditions), respectively.

Any change of the FWHM below said values means that upon changing conditions, starting from non-denaturing conditions, the new conditions are to be subsumed under the term "non-denaturing".

In a particularly preferred embodiment, the term "non-denaturing" applies to relative increases of the FWHM which are below 20%.

In a further preferred embodiment, said physicochemical conditions in accordance with the invention are one, more or all of: (i) pH value; (ii) temperature; (iii) concentration of at least one organic solvent; (iv) concentration of at least one salt; (v) concentration of at least one surfactant; and (vi) concentration of said protein(s), polypeptide(s) and/or peptide(s).

In a particularly preferred embodiment, the change of pH in step (a) is towards the isoelectric point of the protein(s), polypeptide(s) and/or peptide(s) to be precipitated on said inhomogeneous surface or solid particulate matter. It is known that proteinaceous molecules are least soluble in the vicinity of their isoelectric point (IEP). It is also preferred to establish, upon said first change, a pH from 8 to 11, such as from 8 to 10 or from 8 to 9.

Agents which change pH are not particularly limited and include acids and bases.

Preferred acids include inorganic acids such a boric acid, sulfuric acid, nitric acid and hydrogen halides including HF, HCl and HBr; sulfonic acids such as methane sulfonic acid and benzene sulfonic acid; carboxylic acids such as formic acid, acetic acid and citric acid; halogenated carboxylic acids such as trifluoro acetic acid, chloroacetic acid and fluoroacetic acid.

Preferred bases include metal oxides, metal hydroxides, metal carbonates and metal bicarbonates. Particularly preferred bases are NaOH, KOH, LiOH, CsOH, Mg(OH)₂, Ca(OH)₂, Sr(OH)₂, Ba(OH)₂ and furthermore NH₃, tetramethlyammonium hydroxide, guanidine, butyl lithium and NaH.

In a particularly preferred embodiment, the change in temperature in step (a) is a decrease. A decrease of temperature generally decreases solubility which affects different proteinaceous analytes to different extents.

In a particularly preferred embodiment, the change of concentration of an organic solvent is an increase or decrease, preferably of an alcohol such as ethanol, methanol, n-propanol, i-propanol; a nitrile such as acetonitrile; a ketone such as acetone; a glycol ether such as 2-methoxy ethanol; an aliphatic hydrocarbon such as hexane; an aromatic hydrocarbon such as benzene; an aldehyde such as formaldehyde; or mixture thereof.

If the sample to be processed is free or essentially free of organic solvents, an increase of concentration in step (a) is obviously a preferred option.

On the other hand, if the sample to undergo precipitation contains an organic solvent, for example because it has undergone pre-processing involving the addition of an organic solvent, decreasing the concentration of said organic solvent may also be a means of fine-tuning and/or optimizing precipitation conditions.

In a particularly preferred embodiment, the change in concentration of a salt in step (a) is an increase in concentration of said salt, said salt preferably being a kosmotropic salt. "Salting out" using kosmotropic salts is known as a means to trigger precipitation. Preferred kosmotropic salts include ammonium sulfate, potassium phosphate, and salts with a cation selected from , Li+, Zn2+ and Al3+ and an anion selected from carbonate, sulfate and hydrogen phosphate.

It is also envisaged to decrease the concentration of a kosmotropic salt.

In a further particularly preferred embodiment, the change in concentration of a salt in step (a) is a decrease in concentration of said salt, said salt preferably being a chaotropic salt. Chaotropic salts generally increase solubility. Therefore, a decrease in concentration is preferred. Preferred chaotropic salts are NaCl, MgCl₂, guanidinium salts such as guanidinium hydrochloride, perchlorates such as lithium perchlorate, lithium acetate, Barium salts, thiocyanates, iodides and sodium dodecyl sulfate (SDS).

On the other hand, there may be scenarios where a highly abundant protein is prone to precipitation and presence of large amounts thereof in the precipitate are to be avoided. In such a case, adding a chaotropic salt or increasing its concentration in step (a) may be desirable.

The different effect of different salts/cations/anions on precipitation is known in the art as the Hofmeister series.

In a particularly preferred embodiment, the change in concentration of a surfactant is an increase in concentration. Adding a surfactant is a means to keep protein, polypeptides and peptides in solution which otherwise have an undesirably high tendency to precipitate. This applies, for example, to transmembrane proteins such as apolipoproteins.

To the extent said sample comprises surfactants or has undergone pre-processing by adding surfactants, it is also envisaged to decrease the concentration of a surfactant in step (a). This would generally be a means to trigger precipitation.

Preferred surfactants are cholates and deoxycholates such as sodium deoxycholic acid (also referred to as sodium deoxycholate; SDC) and sodium cholate (SC), 2-[4-(2,4,4-trimethylpentan-2-yl)phenoxy]ethanol (Triton X-100), n-dodecyl-β-D-maltopyranoside (DDM), digitonin, Polyoxyethylene (20) sorbitan monolaurate (polysorbate 20), Polyoxyethylene (80) sorbitan monooleate (polysorbate 80), 3-[(3-Cholamidopropyl)dimethylammomo]-1-propanesulfonate (CHAPS), 3-([3-Cholamidopropyl]dimethylammonio)-2-hydroxy-1-propanesulfonate (CHAPSO), polyethylene glycol nonylphenyl ether (NP-40), n-Octyl beta-D-thioglucopyranoside (OTG), (2R,3S,4S,5R,6R)-2-(hydroxymethyl)-6-octoxyoxane-3,4,5-triol (Octyl glucoside), n-decyl-p-D-maltopyranoside (DM), 3-(4-(1,1-bis(hexyloxy)ethyl)pyridinium-1-yl)propane-1-sulfonate (PPS), sodium 3-((1-(furan-2-yl)undecyloxy)carbonylammo)propane-1-sulfonate (ProteaseMAX), or sodium 3-[(2-methyl-2-undecyl-1,3-dioxolan-4-yl)methoxy]-1-propanesulfonate (RapiGest SF); and mixtures thereof.

Further surfactants which may be employed include:
Octyl maltoside, fluorinated
ANAPOE^{®} NID-P40
1,2-Diheptanoyl-sn-Glycero-3-Phosphocholine
Big CHAP, deoxy
C12E8
ANAPOE^{®}-C10E9
CYCLOFOS^{®}-4
CYGLU-3
CYMAL^{®}-6
n-Dodecyl-β-D-maltoside
Dimethyldodecylphosphine oxide
FOS-Choline^{®}-10
HECAMEG
n-Hexyl-β-D-glucoside
MEGA-10
n-Decyl-a-D-maltoside
DDMAB
n-Nonyl-β-D-glucoside
n-Octyl-β-D-glucoside
CHAPS
n-Undecyl-N,N-Dimethylamine-Oxide
TWEEN^{®} 80
n-Decanoylsucrose
ZWITTERGENT^{®} 3-08
1,2-dioctanoyl-sn-glycero-3-phosphocholine
2,6-Dimethyl-4-heptyl-β-D-maltoside
C6E3
C8E5
ANAPOE^{®}-C13E8
CYCLOFOS^{®}-6
CYMAL^{®}-3
Decyl Maltose Neopentyl Glycol
Lauryl Maltose Neopentyl Glycol
FOS-Choline^{®}-12
Genapol^{®} X-100
n-Heptyl-β-D-glucopyranoside
Dimethylnonylphosphine oxide
n-Decyl-N,N-Dimethylamine-Oxide
n-Decyl-β-D-thiomaltoside
n-Heptyl-β-D-thioglucoside
n-Octyl-β-D-maltoside
Nonidet P40
FOS-Choline^{®}-14
C-HEGA-11
ZWITTERGENT 3-16
TRIP AO
ANAPOE^{®}-20
Omega-Undecylenyl-β-D-Maltoside
LysoFos Choline 10
MEGA-9
ANAPOE^{®}-35
C10E5
CHAPSO
CYMAL^{®}-4
CY-TRIPGLU
n-Decyl-β-D-maltoside
Dimethylundecylphosphine oxide
FOS-Choline^{®}-ISO-11
HEGA-10
Façade-EM
LAPAO
n-Decyl-N,N-dimethylglycine
n-Dodecyl-N,N-dimethylglycine
n-Tridecyl-β-D-maltoside
n-Undecyl-β-D-maltoside
Ph-TRIPGLU
Sodium deoxycholate
ZWITTERGENT^{®} 3-10
C8E4
C8E6
ANAPOE^{®}-X-114
ANAPOE^{®}-X-305
C6E4
C7E5
Octyl Glucose Neopentyl Glycol
C12E9
ANAPOE^{®}-C12E10
FOS-Choline^{®}-UNSAT-11-10
CYMAL^{®}-7
MEGA-8
n-decyl-β-D-glucoside
n-Dodecyl-N,N-dimethylamine-N-oxide
n-Dodecyl-a-D-maltoside
n-Nonyl-β-D-maltoside
n-Octyl-β-D-thiomaltoside
n-Undecyl-β-D-thiomaltoside
OHES
1,2-Dihexanoyl-sn-Glycero-3-Phosphocholine
LysoFos Choline 12
RDHPOS
Sodium cholate hydrate
Sodium dodecyl sulfate
ZWITTERGENT^{®} 3-12
n-Dodecyl β-D-glucoside
ANAPOE^{®} X-100
PCC-a-maltoside

Preferred are mild surfactants, i.e., surfactants which do not denaturate proteins, polypeptides and peptides, but solubilize them or portions thereof which are hydrophobic and would aggregate in absence of surfactants. All non-denaturing surfactants among the lists of surfactants given above are preferred. Such surfactants include cholates and deoxycholates such as SDC and SC as well as those given further below.

In a particularly preferred embodiment, the change of concentration of said protein(s), polypeptide(s) and/or peptide(s) in step (a) is an increase. Such increase may be achieved, e.g. by evaporation.

In an alternative particularly preferred embodiment, the change of concentration of said protein(s), polypeptide(s) and/or peptide(s) in step (a) is a decrease, e.g. by adding solvent such as water or buffer (e.g. PBS). Preferably, said solvent to be added mimics the composition of the sample (disregarding said proteins, polypeptides and peptides).

In a further preferred embodiment, said first change of physicochemical conditions is selected from: (i) an increase in pH, preferably to yield a pH value from 8 to 12 such as from 8 to 11 or from 8 to 10, (ii) a rise in temperature, wherein said temperature after said raise is lower than 50°C preferably up to 40°C; (iii) an increase in concentration of an organic solvent, wherein said concentration of said solvent after said increase is less than 20% (v/v); and (iv) an increase in concentration of a chaotropic salt, wherein said concentration of said chaotropic salt after said increase is less than 0.5 M.

Furthermore, an increase in concentration of a surfactant, albeit less preferred, is envisaged.

The above preferred embodiment provides specific definitions of what is more generally referred to as "less harsh" or "non-denaturing" conditions herein.

Particularly preferred temperatures after said raise are less than 40°C, less than 30°C or no raise at all in which case it is preferred to keep said sample at ambient temperature (such as 20°C) or below.

The term "organic solvent" has its art-established meaning. It embraces both protic and aprotic polar organic solvents. In terms of compound classes, it embraces alcohols such as methanol, ethanol and propanol (including i-propanol), aldehydes, ketones such as acetone, and nitriles such as acetonitrile. Further organic solvents are disclosed further above.

Particularly preferred concentrations of said organic solvent are below 15% (v/v), below 10% (v/v), or below 5% (v/v) which includes absence of organic solvents.

Preferred chaotropic salts are disclosed further above. Particularly preferred concentrations of said chaotropic salts are below 0.4 M, below 0.3 M, below 0.2 M, below 0.1 M, below 0.05 M or below 0.01 M, including the absence of chaotropic salts.

Within surfactants or detergents, a distinction can be made as regards their effect on structural and functional integrity of protein, polypeptides and peptides. Denaturing surfactant include sodium dodecyl sulfate (SDS). A further denaturing surfactant is ethyl trimethyl ammonium bromide.

Non-denaturing surfactants include NP40, Triton X-100, Triton X-114, Brij-35, Brij-68, Tween-20, Tween-80, octyl beta-glycoside, octyl thioglycoside, CHAPS, CHAPSO as well as cholates and deoxycholates such as SC and SDC.

Owing to their less harsh properties, non-denaturing surfactants are employed, preferably with concentrations below 5%, below 4%, below 3%, below 2% or below 1%, all (w/v).

Said first change may also involve the addition of a compound selected from PEG, glycerol and DMSO which act as stabilizing agents.

In a further preferred embodiment, said second change of physicochemical conditions is selected from (i) an increase in the concentration of a surfactant, preferably of a non-denaturing surfactant; (ii) a change in concentration of an organic solvent, wherein the total concentration of organic solvents after said change is less than 20% (v/v); (iii) a change in pH, preferably to yield a pH from 6 to 12, more preferably from 8 to 11, and yet more preferably from 9 to 11 or 10 to 11; (iv) a change in temperature; and/or the addition of a compound selected from PEG, glycerol and DMSO.

Preferred or exemplary agents as given above apply to said second change. This includes surfactants, organic solvents, and agents for changing the pH value.

In a particularly preferred embodiment, (i) said non-denaturing surfactant is selected from sodium deoxycholate (SDC), sodium cholate (SC), octyl thioglucoside (OTG), octyl beta-glucoside, NP40, Triton X-100, Triton X-114, Brij-35, Brij-68, Tween-20, Tween-80, CHAPS and CHAPSO; (ii) in case said surfactant is a denaturing surfactant, the concentration of said denaturing surfactant after said increase is below 0.5% (w/v); or (iii) said organic solvent is different from the organic solvent chosen for said first change.

As stated above, cholates and deoxycholates such as SC and SDC are particularly preferred non-denaturing surfactants, especially for said second change. It is preferred that no surfactant is used when performing said first change.

PEG, glycerol and DMSO provide solubilizing and/or stabilizing activity. Generally, the addition of protein-stabilizing agents such as those mentioned here is preferred in step (c).

In a further preferred embodiment, reducing agents are not used or, if used, in concentrations below 0.1% (w/v). Reducing agents include Tris(2-carboxyethyl)phosphine (TCEP) and dithiothreitol (DTT).

In a further preferred embodiment, said solid particulate matter is a plurality of microparticles with a diameter between 0.4 and 500 µm, wherein preferably said plurality of microparticles is identical in composition. More preferred size ranges are from 0.5 to 200 µm, 1 to 100 µm, or from 2 to 50 µm such 3, 4, 5, 6, 7, 8, 9, 10, 15, 20 or 25 µm. Particle sizes in a population of particles are generally distributed around a mean. The above figures refer to said mean. Particle sizes can be determined with art-established methods such as scanning electron microscopy (SEM) or static light scattering (SLS).

In a preferred embodiment, the amount of particles to be used is in the range from 0.01 to 100 µg particles per µl sample, more preferably between 0.1 to 10 µg/µl or between 1 and 5 µg/µl such as 2.5 µg/µl.

In another preferred embodiment, 0.1 to 100 µg protein per µg beads are used such as 1 to 10 or 2 to 5 µg protein per µg beads.

In a further preferred embodiment, wherein said rough surface is etched or porous.

Generally speaking, and this applies to both said solid particulate matter as well as to said inhomogeneous surface, preferably none of these is derivatized or functionalized in order to provide a surface which would preferentially bind certain analytes or classes of analytes. This is different from many prior art approaches which employ tailored surfaces for each analyte to be enriched or depleted. Indeed, there is no requirement for binding moieties on said surface or said particulate matter. Instead, the effect of said surface and said particulate matter is that of a seed. Seeding is a measure to trigger a change of state of matter which change of matter, in the absence of a seed, would not occur, occur delayed or would require harsher physicochemical conditions to occur.

In a further preferred embodiment, said solid particulate matter consists of or comprises one, more or all of (i) a magnetic or magnetizable composition; (ii) floating particles; and (iii) sedimenting particles. These various types of particles may be used as a single type of particle when working the method of the invention, but also allow for embodiments where more than one type of particulate matter is used, thereby generating a plurality of first fractions or precipitates.

For the ease of processing by magnetic means, magnetic or magnetizable compositions such as paramagnetic beads or particles such as silica-coated paramagnetic beads or particles are preferred.

In a further preferred embodiment, said solid particulate matter consists of or comprises polypropylene (PP), polystyrene (PS), polystyrene divinyl benzene (PS-DVB), poly-tetrafluoro ethylene (PTFE), poly-vinyl chloride (PVC), polyoxymethylene (POM), polyethylene (PE) including high-density polyethylene (HDPE) and low-density polyethylene (LDPE), polyamide (PA), polycarbonate (PC), polyethylene terephthalate (PET), polymethyl methacrylate (PMMA), polybutylene terephthalate (PBT), acrylonitrile butadiene styrene (ABS), silica, silanol, ceramics, glass, or metal.

The materials preferred for said solid particulate matter are also preferred vessel materials, said vessel being a vessel with a rough surface according to the present invention.

In a further embodiment, said solid particulate matter carries defined moieties, preferably (i) hydrophobic moieties such as C4, C8, C18 and styrene; and/or (ii) hydrophilic moieties such as hydroxyl, carboxyl, sulfonyl, secondary, tertiary and quaternary amine, preferably hydroxyl, more preferably silanol.

Of note, while such type of functionalized or derivatized particles is envisaged, there is no requirement in accordance with the invention to use them.

The above-disclosed particles are available from various manufacturers and can be prepared by following guidance provided in the literature, e.g. Hench & West, Chem. Rev. 90,33-72 (1990); Rahman & Padavettan, J. Nanomaterials, article ID 132424 (2012); Rao et al., J. Colloid and Interface Sci. 289, 125-131 (2005); and Rahman et al., Colloids and Surface A 294,102-110 (2007).

The following preferred embodiments of the method of the first aspect relate to preferred downstream processing steps which in turn may precede an analytical method further downstream. A preferred downstream analytical method is MS (typically to be performed after one, more or all processing steps of the following embodiments).

In particular, said fractionating is preferably followed by cleaving the protein(s), polypeptide(s) and/or peptide(s) in one, more or all of said second, third and fourth fraction, preferably with a protease, with a chemical, or mechanically, preferably followed by analyzing the products of said cleaving by mass spectrometry (MS).

More specifically, optional further processing steps are (d) separating the supernatant from the precipitate; (e) washing the precipitate, and optionally combining the washing solution with said supernatant; and (f) cleaving the protein(s), polypeptide(s) and/or peptide(s) in the supernatant of (d) or (e), preferably with a protease, with a chemical, or mechanically. This embodiment focuses on processing further the supernatant where desired.

Given that the precipitate is fractionated further to yield the mentioned third and fourth fractions, it is preferred to process one or both of these fractions further such that eventually the results can be fed into MS.

In that respect it is envisaged to perform one or more of the following steps: (g) separating the third fraction from the fourth fraction; (h) washing the third fraction; (i) solubilizing or resuspending protein(s), polypeptide(s) and/or peptide(s) from said third fraction; and (j) cleaving the protein(s), polypeptide(s) and/or peptide(s) in the solubilisate obtained in step (i), preferably with a protease, with a chemical, or mechanically.

Of note, solubilizing in accordance with step (i) is distinct from solubilizing in accordance with step (c). While step (i) aims at complete or substantially complete solubilization in order to capture all analytes in the third fraction, step (c) is designed to provide partial solubilization such that non-empty third and fourth fraction are obtained and the complexity of the sample is further reduced.

As regards the fourth fraction, optional further processing steps are (k) separating the fourth fraction from the third fraction; (l) washing the third fraction, and optionally combining the washing solution with said fourth fraction; and (m) cleaving the protein(s), polypeptide(s) and/or peptide(s) in the results of (k) or (1), preferably with a protease, with a chemical, or mechanically.

In a particularly preferred embodiment, wherein use is made of said solid particulate matter, said separating is effected by centrifugation, filtration or by magnetic means.

In a particularly preferred embodiment, wherein use is made of said rough surface, said separating is effected by removing the supernatant such as by pipetting, draining or decanting.

In a further particularly preferred embodiment, said solubilizing or resuspending of step (i) is performed with a detergent, a solvent such as trifluoro ethanol (TFE), a chaotropic agent such as urea or thiourea, an acid such as trifluoro acetic acid (TFA), a base such as NaOH, or a mixture thereof.

Preferred surfactants are disclosed herein above.

In a further particularly preferred embodiment, (i) said protease is selected from serine proteases, serine carboxy proteases, cysteine proteases, aspartic proteases, metalloproteases and metallocarboxy proteases, preferably from trypsin, chymotrypsin, Lys-C, Lys-N, Asp-N, Glu-C and IdeS; (ii) said chemical is HCl, BrCN, BNPS-skatole, formic acid, hydroxylamine, or 2-nitro-5-thiocyano benzoic acid; or (iii) mechanical cleaving is by means of at least one moving magnet present in said supernatant or eluate, respectively.

Having regard to proteases, the recited classes are EC classes as follows: serine proteases (EC 3.4.21), serine carboxy proteases (EC 3.4.16), cysteine proteases (EC 3.4.22), aspartic proteases (EC 3.4.23), metalloproteases (EC 3.4.24), and metallocarboxy proteases (EC 3.4.17).

Having regard to mechanical cleavage, preferably by means of a moving magnet, reference is made to the present inventors' earlier patent applications WO 2020/002577 and EP patent applications 20174469, 20174484, and 20179317, herewith incorporated by reference in their entirety.

In brief, mechanical cleavage may be effected by a method of cleaving at least one covalent bond in at least one molecule, wherein said method comprises (a) colliding at least one magnetic body with said at least one molecule; and/or (b) triggering collision of at least one non-magnetic particle with said at least one molecule by moving said at least one magnetic body.

Preferred molecules are biomolecules, in particular proteins, polypeptides and peptides.

Said at least one magnetic body may be implemented as a single magnet, i.e., a piece of ferri-, ferro- or paramagnetic material. Said magnetic body preferably performs a fluctuating or oscillating motion. Such motion is preferably triggered by a fluctuating or oscillating magnetic field. The magnetic field may be generated by an electric current which in turn fluctuates or oscillates. The amperage of the current as a function of time is preferably a rectangular function. The electric current preferably flows through a coil surrounding the vessel where the mechanical cleavage is to occur. Said vessel contains the sample to be processed and said at least one magnetic body.

In a further preferred embodiment, fractions obtained by the method of the invention are subjected to MS, optionally with one, more or all of the intervening processing steps as disclosed above effected before MS. Suitable MS systems or mass spectrometers include (grouped according to the analyzer):
Time-of-flight (TOF) analyzer such as TIMS-TOF Pro (Bruker) or Synapt XS (Waters), 6230B (Agilent), TripleTOF 6600 (Sciex), LCMS-9030 (Shimadzu);
Orbitrap Analyzer such as Q Exactive Orbitrap series, Orbitrap Tribrid, Orbitrap Exploris series (Thermo Fisher Scientific);
Quadrupole analyzer such as Altis (Thermo Fisher Scientific), Evoq (Bruker), 6400 series (Agilent), QTRAP (Sciex), SCIEX Triple Quad 7500 (Sciex), Xevo series (Waters), LCMS-8060 series (Shimadzu); and
Ion Trap analyzer such as LTQ XL (Thermo Fisher Scientific), amazon series (Bruker), FT-ICR (Fourier Transform-Ion cyclotron resonance) analyzer such as solariX (Bruker) or LTQ FT Ultra (Thermo Fisher Scientific).

Preferably, a liquid chromatography (LC) device, more preferably a micro- or nano-flow LC system, is coupled to the mass spectrometer, preferably online.

In a second aspect, the present invention relates to a kit comprising or consisting of (i) particulate matter, preferably as defined above; (ii) a binding buffer capable of establishing a first change of physicochemical conditions as defined above; (iii) a solubilizing buffer capable of establishing the second change of physicochemical conditions as defined above; and, optionally (iv) a washing buffer to be used to wash said particulate matter after adding said binding buffer and prior to adding said solubilizing buffer.

As an alternative or in addition to said particulate matter, said kit may comprise a vessel with a rough surface as disclosed herein.

Preferred embodiments of the method of the first aspect, to the extent applicable, define preferred embodiments of the kit of the second aspect.

As such, a preferred particulate matter are paramagnetic particles such as silica-coated paramagnetic particles.

In a preferred embodiment, said binding buffer has a pH from 5 to 12, preferably from 8 to 11 such as from 8 to 10 or 8 to 9, and/or said solubilizing buffer has a pH from 6 to 12, preferably from 8 to 11 such as from 9 to 11 or 10 to 11.

It is furthermore preferred that said binding buffer and/or said solubilizing buffer are non-denaturing.

It is furthermore preferred that said binding buffer does not contain a surfactant.

It is furthermore preferred that said solubilizing buffer contains a non-denaturing surfactant as defined above. Preferred concentrations of said non-denaturing surfactant are from 0.1 to 10% (w/v) including 0.5 to 5% (w/v) and 1 to 2% (w/v). Particularly preferred non-denaturing surfactants such as cholates and deoxycholates including SC and SDC, preferably in a concentration of 1% (w/v). SDC is especially preferred.

In a preferred embodiment, the washing buffer and the binding buffer are identical. In a further preferred embodiment, binding buffer and solubilizing buffer differ only in that the solubilizing buffer contains a surfactant as defined above while the binding buffer does not.

The Figures show:

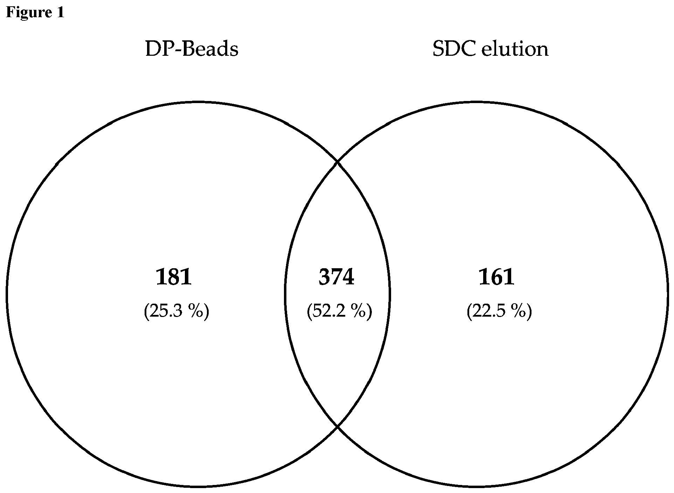

**Figure 1****:** Venn diagram of proteins counts observed when applying the method of the invention.

The Example illustrates the invention.

### Example 1

### Materials and methods

If not otherwise indicated, use is made of the iST procedure (Kulak et. al., Minimal, encapsulated proteomic-sample processing applied to copy-number estimation in eukaryotic cells. Nat Methods 11, 319-324 (2014); incorporated by reference) utilizing the iST buffers from the iST-BCT kit which can be bought from PreOmics GmbH.

250 µg of 3 µm paramagnetic beads are washed in ultrapure water (1 min, shaking at 1200 rpm) and then recovered by magnetic separation. This washing step is performed three times. Afterwards, the beads are mixed with 160 µL of a pH 8 binding buffer.

Starting material is human blood plasma, obtained by collection in K2EDTA anticoagulant tubes. Samples were centrifugated at room temperature at 2000 g for 10 min, and stored frozen afterwards. After thawing to room temperature, 40 µL plasma are added to the beads. The suspension is shaken for 30 min at 1200 rpm at room temperature.

The supernatant is removed by magnetic separation, and the beads are washed three times by adding 200 µL binding buffer for 1 min at 1200 rpm.

For solubilizing proteins, 1% (w/v) SDC in pH 8 buffer (solubilizing buffer) is used. 50 µL of this solution is added to the beads and shaken for 5 min at 500 rpm at room temperature. The supernatant is removed by magnetic separation.

Both the beads as well as the supernatant are purified by the iST-BCT procedure in accordance with the instructions provided by the manufacturer.

The samples are analyzed in triplicates on a nano-LC coupled to a Bruker timsTOF pro mass spectrometer in DIA acquisition mode. Samples are processed in a library-free approach and proteins are required to be present in 2/3 of the biological replicates per condition.

Figure 1 shows a Venn diagram of protein counts observed when applying the method of the invention. The "DP-Beads" (DP stands for "deep plasma" owing to the depth observed) and "SDC elution" workflows were each performed in three replicates and only proteins with valid intensity values in at least two replicates per workflow group were accepted. Data was collected in DIA mode and processed in a library-free approach using the software DIA-NN version 1.8.

### Results

About 500 proteins could be observed in the bead fraction, depending on the blood donor and plasma quality. Analyzing the supernatant increases the protein count by about 30 % as compared to performing only steps (a) and (b) of the method of the first aspect. Protein counts found in the fractions and their overlaps are displayed in Figure 1.

## Claims

1. A method of fractionating a sample comprising proteins, polypeptides and/or peptides, said method comprising
(a) performing a first change of physicochemical conditions of said sample, wherein said first change establishes precipitating conditions for said proteins, polypeptides and/or peptides, but does not establish denaturing conditions for said proteins, polypeptides and/or peptides; and
(b) performing one or both of the following (i) and (ii):
(i) adding solid particulate matter to said sample; and
(ii) performing said method in a vessel with a rough surface;
wherein steps (a) and (b) can be effected concomitantly or in any order and
yield a first fraction of proteins, polypeptides and/or peptides as a first precipitate on said particulate matter and/or on said inhomogeneous surface, and a second fraction of proteins, polypeptides and/or peptides remaining in a supernatant; and
(c) subjecting said first precipitate to a second change of physicochemical conditions which are capable of solubilizing a part of said first precipitate, thereby obtaining a third fraction remaining precipitated and a fourth fraction in solution.

2. The method of any one of the preceding claims, wherein said physicochemical conditions are one, more or all of:
(i) pH value;
(ii) temperature;
(iii) concentration of at least one organic solvent;
(iv) concentration of at least one salt;
(v) concentration of at least one surfactant, preferably of a non-denaturing surfactant; and
(vi) concentration of said protein(s), polypeptide(s) and/or peptide(s).

3. The method of claim 1 or 2, wherein said first change of physicochemical conditions is selected from:
(i) an increase in pH, preferably to yield a pH value from 8 to 11, more preferably a pH value from 8 to 10;
(ii) a raise in temperature, wherein said temperature after said raise is lower than 50°C;
(iii) an increase in concentration of an organic solvent, wherein said concentration of said solvent after said increase is less than 20% (v/v); and
(iv) an increase in concentration of a chaotropic salt, wherein said concentration of said chaotropic salt after said increase is less than 0.5 M.

4. The method of any of the preceding claims, wherein said second change of physicochemical conditions is selected from
(i) an increase in the concentration of a surfactant, preferably of a non-denaturing surfactant;
(ii) a change in concentration of an organic solvent, wherein the total concentration of organic solvents after said change is less than 20% (v/v);
(iii) a change in pH, preferably to yield a pH from 6 to 12, more preferably from 8 to 11, and yet more preferably from 9 to 11;
(iv) a change in temperature; and/or
(v) the addition of a compound selected from PEG, glycerol and DMSO.

5. The method of claim 4, wherein
(i) said non-denaturing surfactant is selected from deoxycholates such as sodium deoxycholate (SDC), cholates such as sodium cholate (SC), octyl thioglucoside (OTG), octyl beta-glucoside, NP40, Triton X-100, Triton X-114, Brij-35, Brij-68, Tween-20, Tween-80, octyl beta-glycoside, octyl thioglycoside, CHAPS and CHAPSO;
(ii) in case said surfactant is a denaturing surfactant, the concentration of said denaturing surfactant after said increase is below 0.5% (w/v); or
(iii) said organic solvent is different from the organic solvent chosen for said first change.

6. The method of any one of the preceding claims, wherein said sample is a cell lysate or a bodily fluid, preferably plasma, wherein optionally said cell lysate or said bodily fluid has been treated with an anticoagulant.

7. The method of any one of the preceding claims, wherein said physicochemical conditions upon said first change and/or said second change do not include temperatures above 70°C, total concentrations of denaturing surfactants such as SDS of or above 0.8% (w/v), and total concentrations of organic solvents such as acetonitrile of or above 20% (v/v).

8. The method of any one of the preceding claims, wherein said solid particulate matter is a plurality of microparticles with a diameter between 0.4 and 500 µm, preferably between 1 and 10 µm , wherein preferably said plurality of microparticles is identical in composition.

9. The method of any one of the preceding claims, wherein said rough surface is etched or porous.

10. The method of any one of the preceding claims, wherein said solid particulate matter consists of or comprises one, more or all of
(i) a magnetic or magnetizable composition, preferably paramagnetic beads such as silica-coated paramgnetic beads;
(ii) floating particles; and
(iii) sedimenting particles.

11. The method of any one of the preceding claims, wherein said solid particulate matter or said rough surface consists of or comprises polypropylene (PP), polystyrene (PS), polystyrene divinyl benzene (PS-DVB), poly-tetrafluoro ethylene (PTFE), poly-vinyl chloride (PVC), polyoxymethylene (POM), polyethylene (PE) including high-density polyethylene (HDPE) and low-density polyethylene (LDPE), polyamide (PA), polycarbonate (PC), polyethylene terephthalate (PET), polymethyl methacrylate (PMMA), polybutylene terephthalate (PBT), acrylonitrile butadiene styrene (ABS), silica, silanol, ceramics, glass, or metal.

12. The method of any one of the preceding claims, wherein said solid particulate matter carries
(i) hydrophobic moieties such as C4, C8, C18 and styrene; and/or
(ii) hydrophilic moieties such as hydroxyl, carboxyl, sulfonyl, secondary, tertiary and quaternary amine, preferably hydroxyl, more preferably silanol.

13. The method of any one of the preceding claims, wherein said fractionating is followed by cleaving the protein(s), polypeptide(s) and/or peptide(s) in one, more or all of said second, third and fourth fraction, preferably with a protease, with a chemical, or mechanically, preferably followed by analyzing the products of said cleaving by mass spectrometry (MS).

14. A kit comprising
(i) particulate matter, preferably as defined in any one of claims 10 to 12;
(ii) a binding buffer capable of establishing a first change of physicochemical conditions as defined in any one of claims 1 to 3 and 7;
(iii) a solubilizing buffer capable of establishing the second change of physicochemical conditions as defined in any one of claims 1, 2, 4, 5 and 7; and, optionally
(iv) a washing buffer to be used to wash said particulate matter after adding said binding buffer and prior to adding said solubilizing buffer.

15. The kit of claim 14, wherein said binding buffer has a pH from 5 to 12, preferably from 8 to 11, and/or said solubilizing buffer has a pH between has a pH from 6 to 12, preferably from 8 to 11.
